# EUROPEAN PATENT APPLICATION

(11) **EP 4 835 905 A1**
(43) Date of publication of application: **04.11.2026**
(21) Application number: 26172682.2
(22) Date of filing: 16.04.2026
(51) Int. Cl.: G16H 50/20, G16H 80/00, G16H 40/63, G16H 40/67, G16H 50/30

(54) **USER DEVICES WITH AI COMPONENTS TO TAKE HEALTH ACTIONS**

(30) Priority: 30.04.2025 US 202519194884
(71) Applicant: T-Mobile USA, Inc., Bellevue, WA 98006-1350 (US)
(72) Inventor: SWAMINATHAN, Kavitha, Bellevue, 98006 (US); BAILEY, Christopher, Bellevue, 98006 (US)
(74) Representative: Suddaby, Mark Edward

(57) **Abstract**

Described herein is an artificial intelligence (AI) component configured to take health actions for a user based on user health information from one or more sensors. In some examples, the AI component is an AI agent that receives the health information, provides the health information in a prompt to a large language model (LLM), and takes a health action recommended by the LLM. In further examples, the AI component determines that the user is experiencing a health emergency and, based on the user not responding to the alerting within a time period, on a health emergency type, and on a user location, alerts an emergency service provider or emergency contact. In additional examples, the AI component determines that the user is at health care provider location for a physical examination and provides a health summary for the user generated for a time period since a last physical examination.

## Description

### BACKGROUND

With each new generation, user devices, such as mobile devices and Internet-of-Things (IoT) devices (e.g., smart watches) are able to measure more and more user health metrics. IoT devices now measure heartrate, blood oxygen levels, and blood pressure. Based on these and other sensed data, IoT devices can even perform analyses such as electrocardiograms.

At the same time as these developments, artificial intelligence (AI) has grown in its abilities from basic machine learning (e.g., determining weights in a model) to acting as an agent that makes decisions and takes actions.

### BRIEF DESCRIPTION OF THE DRAWINGS

The detailed description is set forth with reference to the accompanying figures. In the figures, the left-most digit(s) of a reference number identifies the figure in which the reference number first appears. The same reference numbers in different figures indicate similar or identical items.
FIG. 1 is an overview diagram showing a user device with an artificial intelligence (AI) agent configured to receive health information from sensors about at least one of user health metrics (e.g., blood pressure) or user activities (e.g., visiting a doctor) and to take a health action in response (e.g., alerting an emergency service provider, alerting an emergency contact, providing a health summary to a physician office, etc.), the health action recommended, in some examples, by a large language model (LLM) of the AI agent.
FIG. 2 is a flow diagram of an illustrative process for an AI agent to receive user health information from sensors of a user device, provide the health information in a prompt to a large language model (LLM), and take a health action recommended by the LLM.
FIG. 3 is a flow diagram of an illustrative process for an AI component to determine that the user is experiencing a health emergency and, based on the user not responding to the to an alert within a time period, on a health emergency type, and on a user location, to alert an emergency service provider or emergency contact.
FIG. 4 is a flow diagram of an illustrative process for an AI component to determine that the user is at health care provider location for a physical examination and to provide a health summary for the user generated for a time period since a last physical examination.
FIG. 5 is a schematic diagram of a user device capable of implementing an AI component or AI agent as described herein.

### DETAILED DESCRIPTION

This disclosure is directed in part to an artificial intelligence (AI) component configured to take health actions for a user based on user health information from one or more sensors of a user device (e.g., a wearable device). In some examples, the AI component may be an AI agent that receives the health information, provides the health information in a prompt to a large language model (LLM), and takes a health action recommended by the LLM. As an AI agent, the AI component 106 would generate its own prompts and take its own actions in response to answers from the LLM. Its action may be continual, periodic, or initiated by one or more types of triggers - such as new health information being produced by the user device or new health information meeting a threshold of materiality. To take health actions, the AI agent (AI component 106) may invoke and take action through other components of the user device, such as communication components, user interface components, etc.

In further examples, the AI component may determine that the user is experiencing a health emergency, cause the user device to alert the user, determine that the user has not responded to the alert within a time period, and cause the user device to alert an emergency service provider or emergency contact selected based on a health emergency type and on a user location. For example, health information indicative of a less severe health emergency where an emergency contact is nearby may simply result in a text message to that emergency contact while a more severe health emergency may result in a call to an emergency service provider. In another example, physicians (e.g., doctors, nurses, physician assistants, etc.) may opt in to receive emergency alerts for other nearby persons (much like a physician on an airplane answering a request for a doctor), and the health emergency determined by the AI component may result in a communication to that physician.

In additional examples, the AI component determines that the user is at health care provider location for a physical examination and provides a health summary for the user generated for a time period since a last physical examination. The health summary may include an occurrence when the user pushed a health situation button on the user device and recorded voice or text impressions of how the user then felt, along with readings captured by sensors of the user device at a time of that occurrence.

The user device may be a wearable device (e.g., an Internet-of-Things (IoT) device) or a user equipment (UE) connected to the wearable device and the one or more sensors may include at least one of a microphone, a camera, a motion sensor, a blood pressure sensor, a heartrate sensor, a blood oxygen sensor, a thermometer, or a glucose monitor. In some examples, the wearable device may be a watch, a ring, glasses or other piece of jewelry or apparel worn on a hand, wrist, arm, head, neck, etc. In other examples, the wearable device may be a medical device (e.g., a glucose monitor) paired to a UE.

In various implementations, an artificial intelligence (AI) component may include a large language model (LLM) that is trained on the user's data or data of other users to enable recognition of health patterns for the user. The AI component may also improve in its determination of health information or health actions through continued monitoring of user data. If one health action produces "better" user data at later measurements than another, the AI component may increasingly take the action with better results. "Better," as used herein with respect to user data, may refer to user data that fits health thresholds or ranges defined by a configuration as optimal for users in general, or for the user in particular.

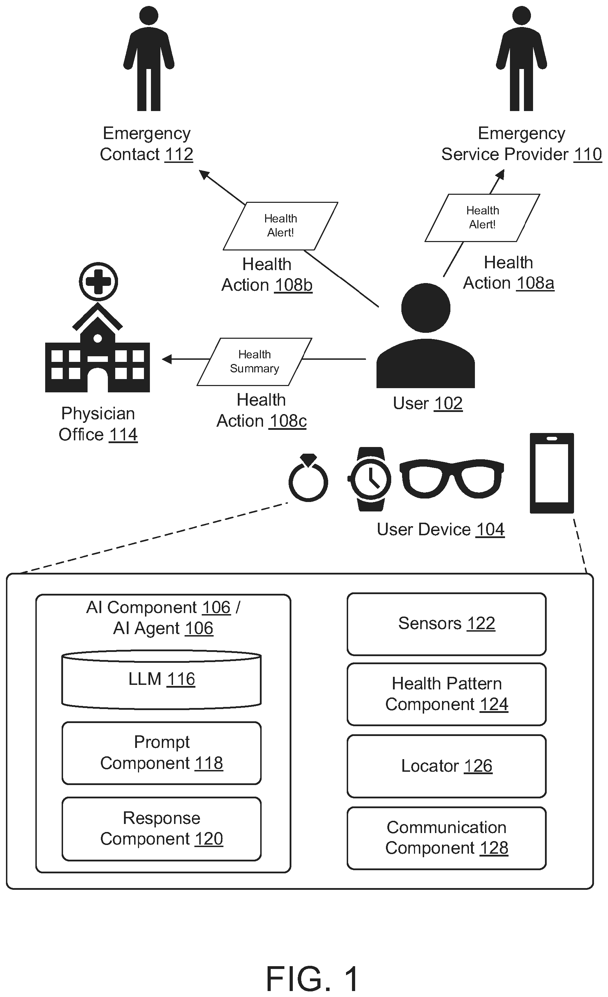

FIG. 1 is an overview diagram showing a user device with an AI agent configured to receive health information from sensors about at least one of user health metrics (e.g., blood pressure) or user activities (e.g., visiting a doctor) and to take a health action in response (e.g., alerting an emergency service provider, alerting an emergency contact, providing a health summary to a physician office, etc.), the health action recommended, in some examples, by an LLM of the AI agent. As illustrated, a user 102 wears a user device 104 that has an AI component 106. The AI component 106 may recommend a health action 108 for the user 102, such as contacting, at 108a, an emergency service provider 110, contacting, at 108b, an emergency contact 112, or providing, at 108c a health summary at a physician's office 114. The AI component 106 may include an LLM 116, a prompt component 118 for generating prompts to submit to the LLM 116, and a response component 120 to recommend a health action 108 based on an answer from the LLM 116. The user device 104 may also have one or more sensors 122 that generate data for health information for the user 102. Further, the user device 104 may have health pattern component 124, a locator 126, and a communication component 128.

In various implementations, the user device 104 may be any sort of wearable devices capable of communication with a UE and/or a telecommunications network. Connections with the UE may be WiFi connections or Bluetooth connections, and a connection with a telecommunications network may be cellular, WiFi, or Bluetooth. To enable such connections, the user device 104 may each have one or more radio antennae for sending and receiving wireless transmission. Further, for cellular transmissions at least, the user device 104 may use any generation of radio access technology. Also, the telecommunications networks mentioned herein may include both terrestrial networks and non-terrestrial networks (e.g., those associated with satellites).

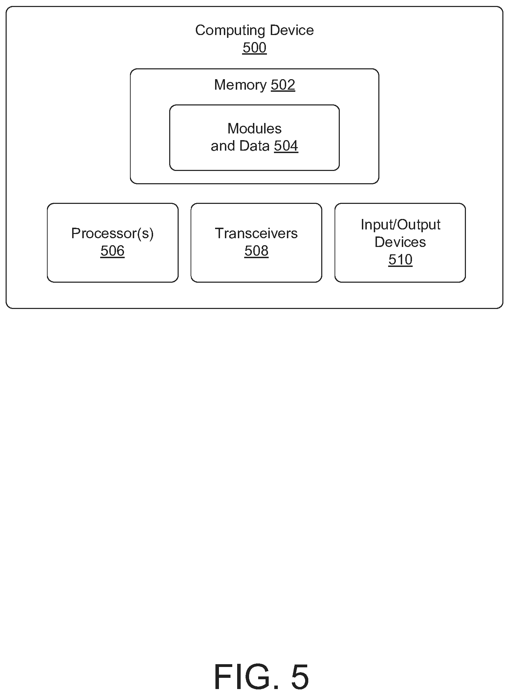

In some examples, such as those shown in FIG. 1, the user device 104 may include a watch (also referred to as a "smart watch") a ring, a necklace, an earring/ ear cuff, glasses, goggles, etc. While user devices 104 as a class may include a wide variety of devices, such as hats, canes, apparel, etc., the user device 104 described herein may be those that have sensor(s) capable of capturing health information such as heartrates, blood oxygen, blood pressure, body temperature, sugar levels, etc. when worn. In various implementations, other user data, such as images or sounds, may also be used to determine the health information. An example user device 104 is illustrated in FIG. 5 and described further herein with reference to that figure.

In various implementations, the user device 104 may include a UE which may be any sort of wireless communications device, such as a cellular phone, a tablet computer, a laptop, etc. In some examples, the UE may have all or part of the AI component 106 as well as some or all of the components 124-128 and may receive health information (or user data on which it is based) from a wearable device of the user device 104. An example UE is illustrated in FIG. 5 and described further herein with reference to that figure.

As shown in FIG. 1, the user device 104 may include an AI component 106 which may be entirely located in the user device 104, distributed between a wearable device and UE comprising the user device 104, or distributed partly locally (e.g., with the user device 104) and partly remotely (e.g., on a remote server or other client device). In some examples, the AI component 106 is an instance of an AI that has instances on multiple devices that share at least the LLM 116. Further, the AI component 106 may be an AI agent 106 or may be part of a program or platform that invokes the AI component 106 and handles processing of any answers from the AI component 106. When the AI component 106 is an AI agent 106, it may be an agentic AI program that operates independently or semi-independently, at least handling the generation of prompts to its LLM 116 and handling answers from the LLM 116. When an agentic AI, the AI agent 106 may operate continuously, respond to periodic triggers, or respond to other triggers, such as new health information.

In various implementations, the LLM 116 may be trained on health information from a general population of users, on health information from a population of users of similar age to the user 102, or on health information from population of users having one or more health conditions that the user 102 has. Such an LLM 116 may be received, for example, from a remote service (e.g., a service associated with a network operator). LLM 116 may receive prompts related to health of the user 102, such as combinations of blood pressure, heart rate, and body temperature, and recommend health actions in response (e.g., "send an ambulance").

In some implementations, the AI component 106 may include a prompt component 118 that responds to triggers to the AI component 106 and generates a prompt to the LLM 116 in response. In some examples, different prompts may be associated with different triggers - e.g., a trigger associated with a new blood pressure measurement may be associated with a base prompt to which other information may be added. For example, all or some recently measured health information from the sensors 122 may be added to the prompt. Also, if a measurement matches/does not match certain criteria and other information may be needed to understand it, the prompt component 118 may utilize a retrieval-augmented generation (RAG) system to retrieve information from one or more external sources (e.g., remote databases) and add that information to the prompt. In addition to adding health information from sensors 122 and/or information from a RAG system, the prompt component 118 may add information from a health pattern component 124 (such as patterns of health information received from the sensors), from a locator 126 (e.g., a current location of the user device 104), or from both.

In further implementations, the response component 120 of the AI component 106 may receive answers from the LLM 116, determine actions to take based on those answers, and take those actions, either directly or through other components of the user device 104 (e.g., through communication component 128). The response component 120 may also learn user preferences - if a user 102 reacts negatively to a health action 108, the response component 120 may learn not to determine a similar health action 108 in the future.

In various implementations, the response component 120 may evaluate health information after taking a health action 108 to determine whether the health information improves relative to health patterns and health standards. When there is no or insufficient improvement, the response component 120 may determine a different health action 108 and monitor and evaluate its effects, too. Over time, the AI component 106 may optimize the health actions chosen for different sorts and magnitudes of health information.

In some examples, the LLM 116 may recommend sending an alert, and the response component 120 may determine, based on user preferences or a current user location to perform one of health action 108a (sending an alert to an emergency service provider 110) or health action 108b (sending an alert to an emergency contact 112). In sending either alert, the response component 120 may invoke the communication component 128 of the user device 104 send the message.

In another example, the prompt to the LLM 116 may indicate that the user 102 has arrived at the physician's office 114 and the response from the LLM 116 may be a health summary. The response component 120, receiving the health summary, may connect via a Bluetooth component of the user device 104 to a device at the physician's office 114 and transmit the health summary as a health action 108c. In another example, there may be a QR code printed in the physician's office, and a QR code reader of the user device 104 may trigger the AI component 106, resulting in the LLM 116 producing a health summary. Further, the generated health summary may include moment(s) when the user 102 did not feel well or felt weird and health metrics (and potentially user dictation) gathered at that/those times.

In some implementations, the health pattern component 124 may receive user data or health information from the sensors, determine health patterns for the user 102, and update those health patterns. Either in response to other logic or to retrieval by the AI component 106, the health pattern component 124 may provide the heath patterns to the AI component 106 for use by the prompt component 118 in forming the prompt.

The locator 126 may be a global positioning system (GPS) sensor or other component configured to determine a location of the user device 104 (e.g., based on access point triangulation, etc.)

The communication component 128 may be or include a calling client or dialer, a messaging client, a video calling client, etc. In some examples, the communication component 128 may be a special purpose component for emergency communications.

In some examples, the communications from the communication component 128 may include health action 108a or 108b. Those health actions 108a or 108b may be calls or messages and may be taken only after first alerting the user 102, may be taken while alerting the user 102, or may be taken without alerting the user 102. If taken after first alerting the user 102, health action 108 may wait for expiration of a timer without a user response. While a response may be something like interacting with a user interface display of an alert, it can also be something like normal movement or conversation with is not consistent with a health emergency. When reaching out to an emergency service provider 110, the communication component 128 may message an on-call physician contact that alerts a physician within a certain proximity of the user 102 with an emergency alert.

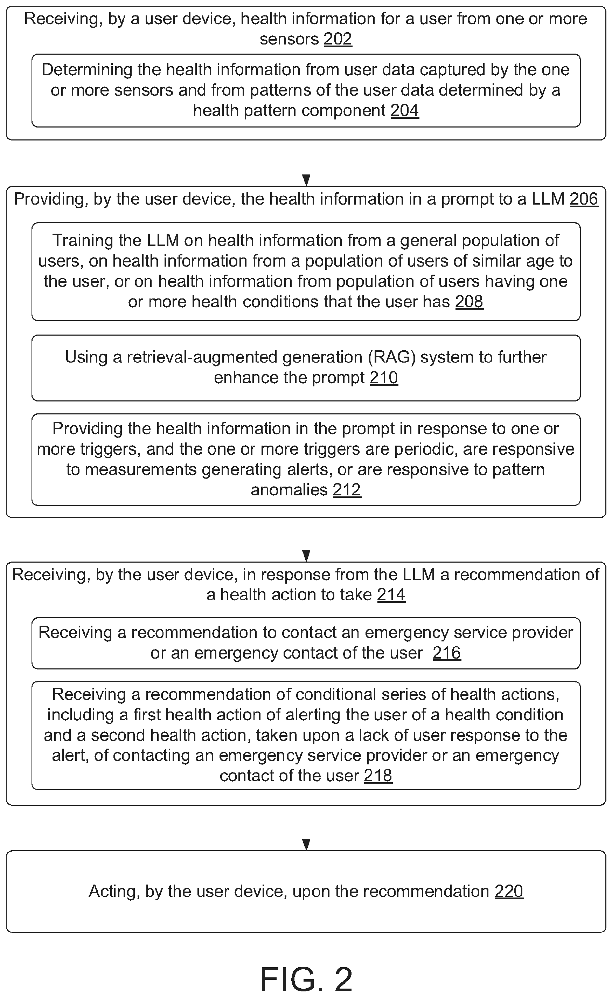

FIGs. 2-4 illustrate example processes. These processes are illustrated as logical flow graphs, each operation of which represents a sequence of operations that can be implemented in hardware, software, or a combination thereof. In the context of software, the operations represent computer-executable instructions stored on one or more computer-readable storage media that, when executed by one or more processors, perform the recited operations. Generally, computer-executable instructions include routines, programs, objects, components, data structures, and the like that perform particular functions or implement particular abstract data types. The order in which the operations are described is not intended to be construed as a limitation, and any number of the described operations can be omitted or combined in any order and/or in parallel to implement the processes.

FIG. 2 is a flow diagram of an illustrative process for an AI agent to receive user health information from sensors of a user device, provide the health information in a prompt to an LLM, and take a health action recommended by the LLM. In some examples, the user device may be at least one of an IoT device or a UE. In some examples, the AI agent may be one instance of a cross-platform AI agent, with multiple instances sharing the LLM and health information from multiple devices.

As illustrated at 202, the AI agent may receive health information for a user from one or more sensors, such as one or more sensors of the user device that at least partly includes the AI agent. The one or more sensors may include at least one of a microphone, a camera, a motion sensor, a blood pressure sensor, a heartrate sensor, a blood oxygen sensor, or a glucose monitor. In some implementations, as shown in 204, a health pattern component of the user device may have determined the health information from user data captured by the one or more sensors and from patterns of the user data determined by the health pattern component as a part of 202.

At 206, the AI agent may provide the health information in a prompt to an LLM. As shown in 208, the LLM may be trained on health information from a general population of users, on health information from a population of users of similar age to the user, or on health information from population of users having one or more health conditions that the user has as a part of 206. As shown in t 210, the AI agent may use a RAG system to further enhance the prompt as a part of 206. As shown in 212, the AI agent may provide the health information in the prompt in response to one or more triggers, and the one or more triggers are periodic, are responsive to measurements generating alerts, or are responsive to pattern anomalies as a part of 206.

At 214, the AI agent may receive in response from the LLM a recommendation of a health action to take. As shown in 216, the received health action recommendation may be contacting an emergency service provider or an emergency contact of the user. In some examples, an identity of the emergency service provider or of the emergency contact may at least partially be determined by a location of the user. As shown in 218, the received health action recommendation may comprise a conditional series of health actions, including a first health action of alerting the user of a health condition and a second health action, taken upon a lack of user response to the alert, of contacting an emergency service provider or an emergency contact of the user.

At 220, the AI agent may act upon the recommendation. For example, the AI agent may cause the user device to generate alerts, initiate communication with another user device or computing device, or trigger another device to perform a task based on the recommendation.

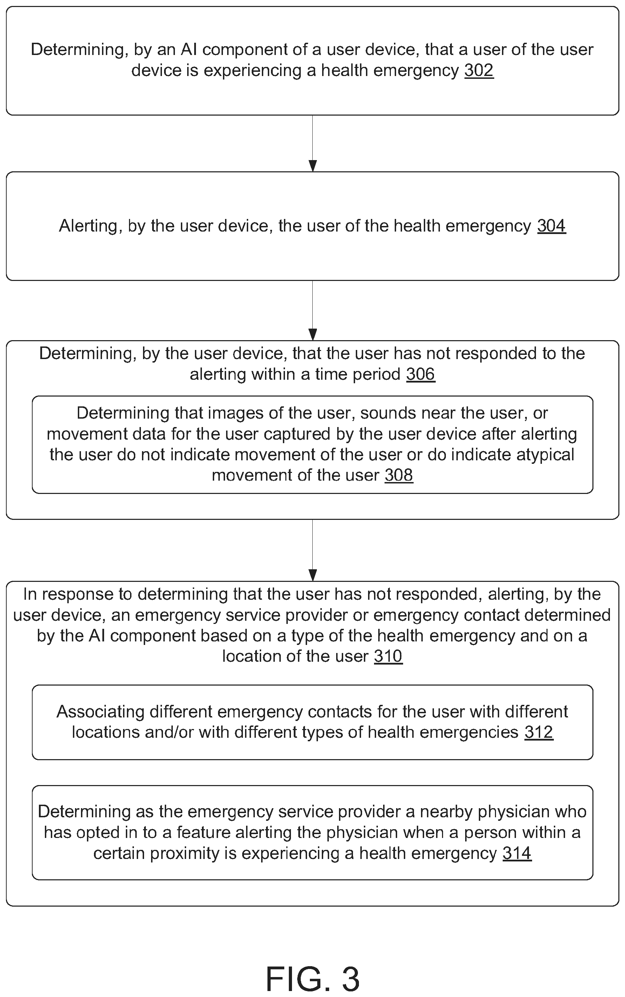

FIG. 3 is a flow diagram of an illustrative process for an AI component to determine that a user is experiencing a health emergency and, based on the user not responding to the to an alert within a time period, on a health emergency type, and on a user location, to alert an emergency service provider or emergency contact. As illustrated at 302, an AI component of a user device may determine that a user of the user device is experiencing a health emergency. The AI component may be an AI agent utilizing an LLM trained on health information from a general population of users, on health information from a population of users of similar age to the user, or on health information from a population of users having one or more health conditions that the user has.

At 304, the user device may alert the user of the health emergency. For example, the alert may be presented visually and/or aurally by a user interface of the user device.

At 306, the user device may determine that the user has not responded to the alerting within a time period. At 308, determining that the user has not responded may include determining that images of the user, sounds near the user, or movement data for the user captured by the user device after alerting the user do not indicate movement of the user or do indicate atypical movement of the user.

At 310, the AI component or other component of the user device may, in response to determining that the user has not responded, alert an emergency service provider or emergency contact. The emergency service provider or emergency contact may be determined by the AI component based on a type of the health emergency and on a location of the user. In some implementations, as shown in 312, the determining by the AI component of the emergency service provider or emergency contact may be based on the AI component associating different emergency contacts for the user with different locations and/or with different types of health emergencies. In other implementations, as shown in 314, the determining by the AI component of the emergency service provider may comprise determining an emergency service provider who is a nearby physician that has opted in to a feature alerting the physician when a person within a certain proximity is experiencing a health emergency.

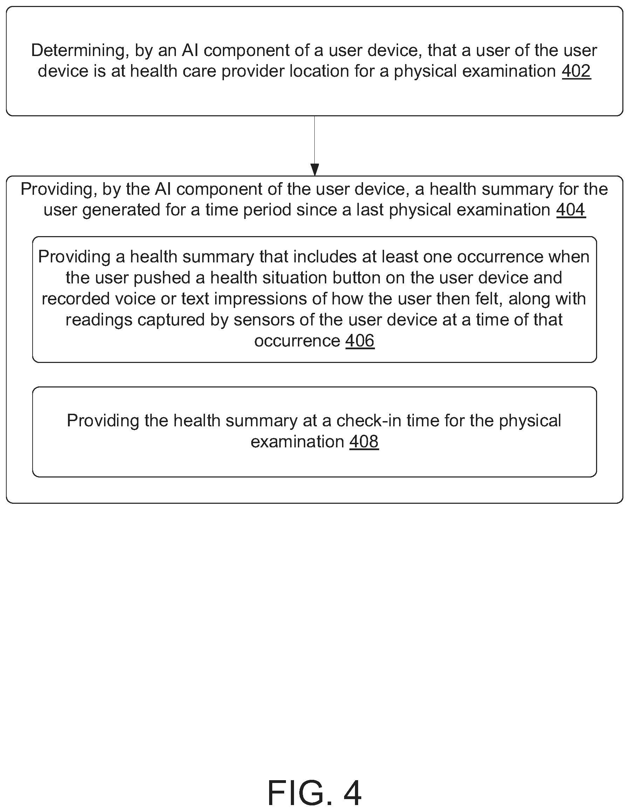

FIG. 4 is a flow diagram of an illustrative process for an AI component to determine that the user is at health care provider location for a physical examination and to provide a health summary for the user generated for a time period since a last physical examination. As illustrated at 402, the AI component of a user device may determine that a user of the user device is at health care provider location for a physical examination. The AI component may be an AI agent utilizing an LLM trained on health information from a general population of users, on health information from a population of users of similar age to the user, or on health information from a population of users having one or more health conditions that the user has.

At 404, the AI component of the user device may provide a health summary for the user generated for a time period since a last physical examination. In some implementations, as shown in 406, the providing may comprise providing a health summary that includes at least one occurrence when the user pushed a health situation button on the user device and recorded voice or text impressions of how the user then felt, along with readings captured by sensors of the user device at a time of that occurrence. In other implementations, as shown in 408, the providing may comprise providing a health summary at a check-in time for the physical examination. In some examples, providing the health summary at the check-in time may comprise providing the health summary over a local wireless connection at the health care provider location.

FIG. 5 is a schematic diagram of a user device capable of implementing an AI component or AI agent as described herein. As shown, the user device 500 includes a memory 502 storing modules and data 504, processor(s) 506, transceivers 508, and input/output devices 510.

In various examples, the memory 502 can include system memory, which may be volatile (such as RAM), non-volatile (such as ROM, flash memory, etc.) or some combination of the two. The memory 502 can further include non-transitory computer-readable media, such as volatile and nonvolatile, removable and non-removable media implemented in any method or technology for storage of information, such as computer readable instructions, data structures, program modules, or other data. System memory, removable storage, and non-removable storage are all examples of non-transitory computer-readable media. Examples of non-transitory computer-readable media include, but are not limited to, RAM, ROM, EEPROM, flash memory or other memory technology, CD-ROM, digital versatile discs (DVD) or other optical storage, magnetic cassettes, magnetic tape, magnetic disk storage or other magnetic storage devices, or any other non-transitory medium which can be used to store the desired information.

The memory 502 can include one or more software or firmware elements, such as computer-readable instructions that are executable by the one or more processors 506. For example, the memory 502 can store computer-executable instructions associated with modules and data 504. The modules and data 504 can include a platform, operating system, and applications, and data utilized by the platform, operating system, and applications. Further, the modules and data 504 can implement any of the functionality for the devices and components described and illustrated herein.

In various examples, the processor(s) 506 can be a central processing unit (CPU), a graphics processing unit (GPU), or both CPU and GPU, or any other type of processing unit. Each of the one or more processor(s) 506 may have numerous arithmetic logic units (ALUs) that perform arithmetic and logical operations, as well as one or more control units (CUs) that extract instructions and stored content from processor cache memory, and then executes these instructions by calling on the ALUs, as necessary, during program execution. The processor(s) 506 may also be responsible for executing all computer applications stored in the memory 502, which can be associated with types of volatile (RAM) and/or nonvolatile (ROM) memory.

The transceivers 508 can include modems, interfaces, antennas, Ethernet ports, cable interface components, and/or other components that perform or assist in exchanging wireless communications, wired communications, or both.

While the computing device need not include input/output devices 510, in some implementations it may include one, some, or all of these. For example, the input/output devices 510 can include a display, such as a liquid crystal display or any other type of display. For example, the display may be a touch-sensitive display screen and can thus also act as an input device or keypad, such as for providing a soft-key keyboard, navigation buttons, or any other type of input. The input/output devices 510 can include any sort of output devices known in the art, such as a display, speakers, a vibrating mechanism, and/or a tactile feedback mechanism. Output devices can also include ports for one or more peripheral devices, such as headphones, peripheral speakers, and/or a peripheral display. The input/output devices 510 can include any sort of input devices known in the art. For example, input devices can include a microphone, a keyboard/keypad, and/or a touch-sensitive display, such as the touch-sensitive display screen described above. A keyboard/keypad can be a push button numeric dialing pad, a multi-key keyboard, or one or more other types of keys or buttons, and can also include a joystick-like controller, designated navigation buttons, or any other type of input mechanism.

Although features and/or methodological acts are described above, it is to be understood that the appended claims are not necessarily limited to those features or acts. Rather, the features and acts described above are disclosed as example forms of implementing the claims.

Also, while the descriptions provided herein may be in the context of certain radio access technologies, networks, and network topologies, such as Fifth Generation (5G)/new radio (NR) mobile communications, the proposed concepts, schemes, and any variations thereof may be implemented in, for and by other types of radio access technologies, networks, and network topologies. Such radio access technologies, networks, and network topologies may include, for example and without limitation, Long-Term Evolution (LTE), Internet-of-Things (IoT), Narrow Band Internet of Things (NB-loT), vehicle-to-everything (V2X), fixed wireless internet, and NTN communications. Thus, the scope of the disclosure is not limited to the examples described herein.

Additional aspects of the present disclosure reside in the following numbered clauses:
Clause 1. A non-transitory computer storage medium having programming instructions stored thereon that, when executed by one or more processors of a user device, cause the user device to perform operations comprising:
   determining, by an artificial intelligence (AI) component of the user device, that a user of the user device is at health care provider location for a physical examination; and
   providing, by the AI component of the user device, a health summary for the user generated for a time period since a last physical examination.
Clause 2. The non-transitory computer storage medium of clause 1, wherein the health summary includes at least one occurrence when the user pushed a health situation button on the user device and recorded voice or text impressions of how the user then felt, along with readings captured by sensors of the user device at a time of that occurrence.
Clause 3. The non-transitory computer storage medium of clause 1 or 2, wherein the AI component is an AI agent utilizing a large language model (LLM) trained on health information from a general population of users, on health information from a population of users of similar age to the user, or on health information from a population of users having one or more health conditions that the user has.
Clause 4. The non-transitory computer storage medium of any of clauses 1 to 3, wherein the providing comprises providing the health summary at a check-in time for the physical examination.
Clause 5. The non-transitory computer storage medium of any of clauses 1 to 4, wherein the providing the health summary at the check-in time comprises providing the health summary over a local wireless connection at the health care provider location.

## Claims

1. A user device comprising:
one or more processors; and
an artificial intelligence, AI, agent configured to be operated by the one or more processors to:
receive health information for a user from one or more sensors;
provide the health information in a prompt to a large language model, LLM; receive in response from the LLM a recommendation of a health action to take; and act upon the recommendation.

2. The user device of claim 1, wherein the user device is at least one of a wearable device or a user equipment, UE, and the one or more sensors include at least one of a microphone, a camera, a motion sensor, a blood pressure sensor, a heartrate sensor, a thermometer, a blood oxygen sensor, or a glucose monitor.

3. The user device of claim 1 or 2, wherein the LLM is trained on health information from a general population of users, on health information from a population of users of similar age to the user, or on health information from population of users having one or more health conditions that the user has.

4. The user device of any preceding claim, further comprising a health pattern component configured to be operated by the one or more processors to determine the health information from user data captured by the one or more sensors and from patterns of the user data determined by the health pattern component.

5. The user device of any preceding claim, wherein the AI agent is configured to use a retrieval-augmented generation, RAG, system to further enhance the prompt.

6. The user device of any preceding claim 1, wherein the AI agent is one instance of a cross-platform AI agent, with multiple instances sharing the LLM and health information from multiple devices.

7. The user device of any preceding claim, wherein the AI agent provides the health information in the prompt in response to one or more triggers, and the one or more triggers are periodic, are responsive to measurements generating alerts, or are responsive to pattern anomalies.

8. The user device of any preceding claim, wherein the health action is contacting an emergency service provider or an emergency contact of the user, wherein an identity of the emergency service provider or of the emergency contact is at least partially determined by a location of the user.

9. The user device of any preceding claim, wherein the health action comprises a conditional series of health actions, including a first health action of alerting the user of a health condition and a second health action, taken upon a lack of user response to the alert, of contacting an emergency service provider or an emergency contact of the user.

10. A method comprising:
determining, by an artificial intelligence, AI, component of a user device, that a user of the user device is experiencing a health emergency;
alerting, by the user device, the user of the health emergency;
determining, by the user device, that the user has not responded to the alerting within a time period; and
in response to determining that the user has not responded, alerting, by the user device, an emergency service provider or emergency contact determined by the AI component based on a type of the health emergency and on a location of the user.

11. The method of claim 10, wherein the AI component associates different emergency contacts for the user with different locations and/or with different types of health emergencies.

12. The method of claim 10 or 11, wherein determining that the user has not responded includes determining that images of the user, sounds near the user, or movement data for the user captured by the user device after alerting the user do not indicate movement of the user or do indicate atypical movement of the user.

13. The method of any of claims 10 to 12, wherein the AI component is an AI agent utilizing a large language model, LLM, trained on health information from a general population of users, on health information from a population of users of similar age to the user, or on health information from a population of users having one or more health conditions that the user has.

14. The method of any of claims 10 to 13, wherein the emergency service provider determined by the AI component is a nearby physician who has opted in to a feature alerting the physician when a person within a certain proximity is experiencing a health emergency.

15. A computer-readable storage medium having programming instructions stored thereon that, when executed by one or more processors of a user device, cause the user device to perform the method of any of claims 10 to 14.
